# EUROPÄISCHE PATENTSCHRIFT

(11) **EP 0 734 682 B1**
(45) Veröffentlichungstag und Bekanntmachung des Hinweises auf die Patenterteilung: **29.09.1999**
(21) Anmeldenummer: 96103303.2
(22) Anmeldetag: 04.03.1996
(51) Int. Cl.: A61B 1/00, A61B 17/28, A61B 17/32, B23K 26/00, B23K 31/10

(54) **Medizinisch-endoskopisches Gerät mit superelastischem Element**
Endoscopic medical device with superelastic element
Dispositif médical endoscopique avec élément superélastique

(30) Priorität: 25.03.1995 DE 19510962
(43) Veröffentlichungstag der Anmeldung: 02.10.1996
(73) Patentinhaber: OLYMPUS WINTER & IBE GmbH, 22045 Hamburg (DE)
(72) Erfinder: Benecke, Rainer, Dr.-Ing., 22965 Todendorf (DE)
(74) Vertreter: Schaefer, Konrad, Dipl.-Phys.

(56) Entgegenhaltungen:
- DE-C- 4 313 903

## Beschreibung

Die Erfindung betrifft ein Gerät der im Oberbegriff des Anspruchs 1 genannten Art.

Aus der Literatur sind einige für die vorliegenden Zwecke geeignete superelastische Legierungen bekannt, wie insbesondere Ni-Ti-Legierungen. Diese Legierungen sind in der Medizin vielfältig verwendbar. Sie sind biokompatibel und weisen einen Formgedächtniseffekt auf, der für viele Zwecke verwendbar ist. Für die vorliegenden Zwecke zeichnen sie sich durch ihre superelastischen Eigenschaften aus, die dazu führen, daß bei Kraftbeaufsschlagung eine Förmänderung bis zu etwa 8 % unter konstanter Kraft möglich ist. Diese Legierungen können also als kraftübertragende Elemente eingesetzt werden, die in erheblichem Maße verformbar sind, ohne zu brechen oder die beispielsweise zur Kraftbegrenzung eingesetzt werden, wie in der
DE 43 13 903 C1
beschrieben.

Dem breiteren Einsatz superelastischer Legierungen für die gattungsgemäßen Zwecke stehen aber fertigungstechnische Probleme im Wege. Nach dem bisher bekanntgewordenen Stand der Technik, wie er in der oben genannten Schrift angegeben ist, ist zu entnehmen, daß superelastische Legierungen zu ihrer Herstellung einer aufwendigen Einstellung des genauen Phasenzustandes bedürfen. Dazu muß die Legierung in ihrer genauen Zusammensetzung stimmen und es ist ein aufwendiger Herstellungsprozeß erforderlich. Nachträgliche konventionelle Bearbeitungen sind kaum möglich.

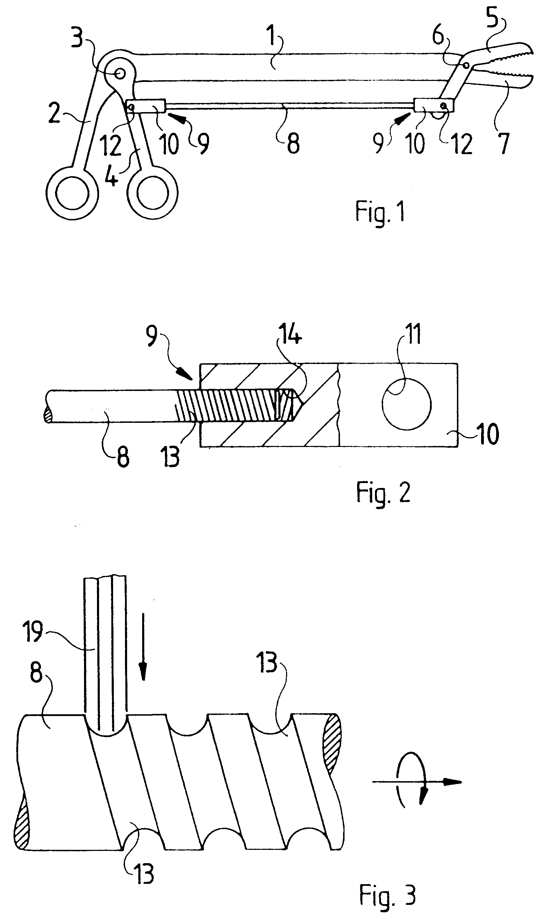

Im Falle der in der oben genannten Schrift beschriebenen Zugstange 6 (Fig. 1) ergeben sich Probleme mit der Kupplung dieses Elementes an seinen Enden, an denen es mit Lastaufnahmegliedern 5, 10 gekuppelt werden muß. Diese Kupplungsstellen lassen sich nach dem Stand der Technik nur herstellen, wenn die Zugstange an ihren Enden mit im Werk vorgefertigten Kupplungsstellen hergestellt und thermisch behandelt wurde. Die Verwendung von Abschnitten von Endlosdraht, die große Kostenvorteile böte, ist ausgeschlossen.

Würde man Abschnitte von Endlosmaterial aus superelastischer Legierung verwenden, so ließen sich diese nicht bearbeiten. Aufgrund der superelastischen Eigenschaften des Materiales ist Kaltverformung oder spanabhebende Bearbeitung unmöglich. Löten mit geeigneter Festigkeit ist bei diesen Legierungen ebenfalls nicht möglich. Schweißen scheidet wegen der Temperaturbelastung aus.

Alle Versuche zu nachträglicher Bearbeitung superelastischer Legierungen bleiben entweder ohne Erfolg oder führen zu einer mechanischen oder thermischen Belastung der Legierung, die deren superelastische Eigenschaften zerstört und dazu führt, daß die Festigkeit des Materials nicht mehr ausreichend ist. Bei späterer Belastung bricht das Material.

Gattungsgemäße kraftübertragende Elemente müssen daher nach dem Stand der Technik in ihrer Endform in dem erforderlichen Prozeß hergestellt werden und können nachträglich nicht mehr bearbeitet werden. Dadurch ergeben sich sehr hohe Fertigungskosten. Die Verwendung billigeren Endlosmateriales ist nicht möglich.

Die Aufgabe der vorliegenden Erfindung besteht darin, ein Gerät der eingangs genannten Art zu schaffen, das kostengünstiger herstellbar ist.

Diese Aufgabe wird erfindungsgemäß mit den Merkmalen des Kennzeichnungsteiles des Anspruchs 1 gelöst.

Überraschend hat sich herausgestellt, daß sich Vertiefungen auf Oberflächen superelastischer Legierungen durch Sublimationsabtragung herstellen lassen, ohne die Materialeigenschaften zu verändern. Damit ist es möglich, solche Vertiefungen vorzusehen, die für Formschlußverbindungen geeignet sind. Es ist daher möglich, Endlosmaterialien aus superelastischen Legierungen zu verwenden und somit die Herstellungskosten gattungsgemäßer Geräte wesentlich zu verringern. Es können z.B. Endlosdrähte oder -rohre aus superelastischer Legierung, zugeschnitten werden und an erforderlichen Kupplungsstellen auf der Oberfläche mit Vertiefungen versehen werden, an denen eine Formschlußverbindung mit Lastaufnahmegliedern mit hoher Festigkeit hergestellt werden können.

Unter Sublimationsabtragung wird die Materialabtragung durch Sublimation verstanden. Der physikalische Begriff "Sublimation" bezeichnet einen Vorgang, bei dem ein Material aus der festen Phase unmittelbar in die Gasphase übergeht, so wie z.B. Wassereis an trockenen kalten Wintertagen unmittelbar aus der Eisphase als Wasserdampf sublimiert. Bei der Sublimation entsteht keine flüssige Phase, wie sonst bei Verdampfüngsvorgängen üblich.

Sublimationsabtragung kann durch geeignete Energiebeaufschlagung des Materials erfolgen, beispielsweise mit Elektronenstrahlen oder dergleichen. Wichtig ist dabei nur, daß stets nur sublimiert wird, also oberste Materialschichten derart rasch aufgeheizt werden, daß sie unmittelbar verdampfen, ohne daß das darunter liegende Material zu stark aufgeheizt und dadurch thermisch beeinträchtigt wird. Das Material wird dabei nicht geschmolzen. Es entsteht also keine flüssige Phase, welche starke Temperaturbeeinflussungen des Materiales und dadurch starke Veränderungen der komplizierten Kristallstruktur der superelastischen Legierung ergeben würde.

Vorteilhaft sind dabei die Merkmale des Anspruches 2 vorgesehen. Die Sublimation mittels Laserbestrahlung hat den Vorteil, daß sie mit geringem apparativen Aufwand, also z.B. ohne Vakuumkammer oder dergl., auskommt. Außerdem lassen sich mittels Laserstrahlbearbeitung äußerst feine Mikrostrukturen erzeugen, wie sie bei sehr fein strukturierten endoskopischen Instrumenten erforderlich sein können.

Vorteilhaft sind die Merkmale des Anspruches 3 vorgesehen. Bei Ausbildung der Vertiefung als Gewinde läßt sich die Formschlußverbindung als Gewindeeingriff herstellen, was üblichen Konstruktionsmethoden entgegenkommt.

Alternativ sind vorteilhaft die Merkmale des Anspruches 4 vorgesehen. Eine solche Quetschverbindung kann hergestellt werden mit Kräften, die lediglich den Formschlußeingriff bewirken, aber nicht das superelastische Material zu stark belasten.

In den Zeichnungen ist die Erfindung beispielsweise und schematisch dargestellt. Es zeigen:
- Fig. 1:: in Seitenansicht eine endoskopische Zange mit superelastischem Kraftübertragungselement mit zwei Kupplungsstellen,
- Fig. 2:: in vergrößerter Teilschnittdarstellung eine der Kupplungsstellen der Figur 1.
- Fig. 3:: in vergrößerter Seitenansicht zu Fig. 2 den Herstellungsvorgang des Gewindes,
- Fig. 4 - 7:: weitere Ausführungsformen von Kupplungsstellen in Ansicht gemäß Fig. 2.
- Fig. 8:: in Achsialschnittdarstellung ein superelastisches Rohr einer Endoskopoptik mit zwei Kupplungsstellen.

Fig. 1 zeigt eine Zange für endoskopische Zwecke in stark schematisierter Darstellung.

Ein Schaft 1 geht an seinem proximalen Ende in einen starr verbundenen Scherengriff 2 über. Ein mit einer Achse 3 angelenkter beweglicher Scherengriff 4 dient zur Steuerung einer beweglichen Branche 5 um eine Achse 6 gegenüber einer mit dem Schaft 1 starr verbundenen festen Branche 7.

Zur Bewegungssteuerung ist als kraftübertragendes Element eine Zugstange 8 aus superelastischer Legierung vorgesehen, die aus vorgefertigtem Endlosdraht als Abschnitt hergestellt ist. An ihren an den Enden vorgesehenen Kupplungsstellen 9 ist die Zugstange 8 mit als Lastaufnahmegliedern dienenden Hülsen 10 verbunden, die jeweils mit einer Querbohrung 11 über Achsen 12 an dem beweglichen Scherengriff 4 bzw. der beweglichen Branche 5 der Zange gelagert sind.

Zur Formschlußverbindung der Kupplungsstelle 9 an den Enden der Zugstange 8 mit der jeweiligen Hülse 10 sind die Kupplungsstellen auf ihrer Oberfläche mit Vertiefungen versehen, die im dargestellten Ausführungsbeispiel als Gewindegänge 13 ausgebildet sind. Diese greifen in eine Bohrung 14 der jeweiligen Hülse 10, die mit passendem Innengewinde ausgebildet ist.

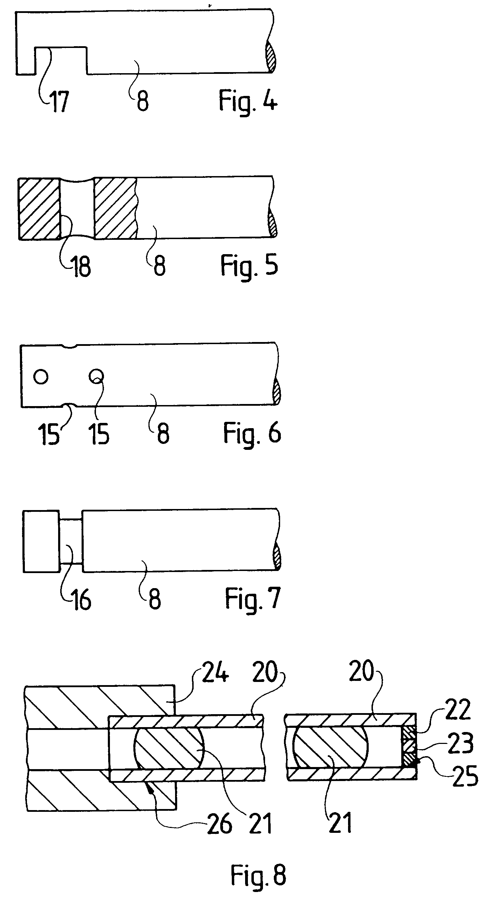

Alternativ kann die Hülse 10 auch auf die Gewindegänge 13 gequetscht werden. In diesem Fall können die Vertiefungen auf der Oberfläche der Kupplungsstelle 9 auch in anderer Weise ausgebildet sein, beispielsweise als Punktvertiefungen 15, wie dies Fig. 6 zeigt, als Umfangsrille 16, wie dies Fig. 7 zeigt, als Quernut 17, wie dies Fig. 4 zeigt oder als durchgehendes Querloch 18, wie dies Fig. 5 zeigt.

Die Herstellung der Vertiefungen 13, 15, 16, 17 oder 18 auf der Oberfläche der Zugstange 8, die aus superelastischem Material besteht, ist mit konventionellen Methoden nicht möglich, da zur Lötung auf superelastischen Legierungen keine geeigneten Lötmittel zur Verfügung stehen, Schweißen wegen zu hoher thermischer Belastung und mechanische Verformung wegen zu hoher mechanischer Belastung der Legierung ausscheidet.

Wie Fig. 3 zeigt, wird die im Ausführungsbeispiel als Gewindegang 13 ausgebildete Vertiefung in der Oberfläche der Zugstange 8 mit einem gebündelten Energiestrahl 19 erzeugt, der in Pfeilrichtung auf die Oberfläche gerichtet ist. Besonders vorteilhaft ist als Energiestrahl 19 ein Laserstrahl verwendbar, mit dem in freier Umgebung, also ohne Vakuumkammer oder dergl. gearbeitet werden kann. Ein Laserstrahl läßt sich durch geeignete Optiken, die in der Zeichnung nicht dargestellt sind, fein fokussieren und mit ausreichender Genauigkeit und Schnelligkeit derart in seiner Energiezuführ steuern, daß nur jeweils die oberste Materialschicht des superelastischen Materiales verdampft wird, das darunterliegende Material aber nicht übermäßig unter Zerstörung der superelastischen Eigenschaften erhitzt wird. Dies kann insbesondere durch gepulste Beaufschlagung mit sehr starken Impulsen erfolgen, die lediglich eine kurzfristige Materialverdampfung bewirken.

Im dargestellten Ausführungsbeispiel der Fig. 3 steht aus apparativen Gründen der Laserstrahl 19 fest, während zur schraubenförmigen Erzeugung des Gewindeganges 13 die Zugstange 8 in Richtung der Pfeile bewegt und gedreht wird.

Die Vertiefungen der Ausführungsformen der Figuren 4 - 7 können im wesentlichen in derselben Weise hergestellt werden, wobei bei Verwendung von mit Optiken fein fokussierten Laserstrahlen sehr feine Strukturen, z.B. gemäß Fig. 7 eine Umfangsrille 16 mit exakt rechteckigem Querschnitt hergestellt werden kann, die sich gut zur Formschlußkupplung mit einem geeignetem Lastaufnahmeglied eignet.

Fig. 8 zeigt eine weitere Ausführungsform der Erfindung, bei der das kraftübertragende Element ein Rohr 20 einer Endoskopoptik ist. In dem Rohr 20 sind die Stablinsen 21 einer üblichen Endoskopoptik angeordnet. Am distalen Ende des Rohres 20 ist mit einem Innenring 22 ein Fenster 23 gehalten. Am proximalen Ende ist das Rohr 20 in der Bohrung eines Endoskophauptkörpers 24 eingesetzt, an dem das nicht dargestellte Okular vorgesehen ist.

Die Verwendung eines Rohres 20 aus superelastischem Material hat für viele medizinische Anwendungsfälle große Vorteile, bei denen das Rohr 20 hohe Biegekräfte übertragen muß. Ein typisches Anwendungsbeispiel sind sehr lange und sehr dünne Optiken, mit denen der Urologe durch den Ureter bis zur Niere geht. Typischerweise sind solche Optiken etwa 40 cm lang bei einem Außendurchmesser von 3 mm. Beim Einführen müssen solche Optiken hohe Biegekräfte aufnehmen, die häufig zur Zerstörung führen, was durch Verwendung superelastischer Materialien vermieden werden kann.

Problematisch ist dabei die ausreichend sichere Befestigung des Rohres an den Kupplungsstellen 25 und 26, die im dargestellten Ausführungsbeispiel der Fig. 8 die Kupplungsstellen mit dem Innenring 22 und dem Hauptkörper 24 sind.

Sichere Verbindungen an diesen Kupplungsstellen lassen sich nur mit Formschlußeingriff herstellen, also beispielsweise durch ein Innengewinde zum Halten des Innenringes 22 an der Kupplungsstelle 25 und ein Außengewinde an der Kupplungsstelle 26 zur Befestigung im Hauptkörper 24. Soll das Rohr 20 nicht werkseitig entsprechend ausgerüstet, sondern aus Endlosrohr hergestellt werden, so können Gewinde oder sonstige Oberflächenvertiefungen zur Formschlußverbindung entsprechend Fig. 3 in Sublimationstechnik hergestellt werden.

Auch die übrigen Schäfte von Endoskopen, beispielsweise ein über dem Rohr 20 verwendeter Endoskopschaft, können aus superelastischem Material hergestellt werden. Die Kupplungsstellen solcher Schäfte an Hauptkörpern, Spülflüssigkeitsventilen oder dergl. können durch Sublimation bearbeitet werden.

Auch weitere kraftübertragende Elemente von Geräten für die medizinische Endoskopie lassen sich auf diese Weise ausführen. So kann beispielsweise bei der endoskopischen Zange der Figur 1 die Branche 5 aus superelastischem Material ausgebildet sein. Die Branche kann durch Lasersublimation aus Endlosplattenmaterial hergestellt werden unter Ausschneiden der Umfangsform und Einbringen der Bohrungen für die Achsen 6 und 12.

## Patentansprüche

1. Gerät für die medizinische Endoskopie, mit einem kraftübertragenden Element (8, 20) aus superelastischer Legierung, das an einer Kupplungsstelle (9, 22, 24), die mit wenigstens einer Vertiefung (13, 15, 16, 17, 18) in der Oberfläche des Elementes ausgebildet ist, mit einem Lastaufnahmeglied (10, 24) durch Formschluß verbunden ist, **dadurch gekennzeichnet**, daß die Vertiefung (13, 15, 16, 17, 18) durch Sublimationsabtragung ausgebildet ist.

2. Gerät nach Anspruch 1, **dadurch gekennzeichnet**, daß die Vertiefung (13, 15, 16, 17, 18) mittels Laserbestrahlung hergestellt ist.

3. Gerät nach Anspruch 1 mit zylinderförmiger Kupplungsstelle (9), **dadurch gekennzeichnet**, daß die Vertiefung (13, 15, 16, 17, 18) als Gewinde (13) ausgebildet ist.

4. Gerät nach Anspruch 1, **dadurch gekennzeichnet**, daß die Formschlußverbindung durch Quetschen des Lastaufnahmegliedes (10) auf die Oberfläche des Elementes (8) ausgebildet ist.

## Claims

1. Device for medical endoscopy with a force-transmitting element (8, 20) of a superelastic alloy, which is form-lockingly connected to a load-receiving member (10, 24) at a coupling point (9, 22, 24), which is formed with at least one recess (13, 15, 16, 17, 18) in the surface of the element, characterised in that the recess (13, 15, 16, 17, 18) in the surface of the element is formed by sublimation erosion.

2. Device as claimed in claim 1, characterised in that the recess (13, 15, 16, 17, 18) is produced by laser irradiation.

3. Device as claimed in claim 1, with a cylindrical coupling point (9), characterised in that the recess (13, 15, 16, 17, 18) is constructed as a screw thread (13).

4. Device as claimed in claim 1, characterised in that the form-locking connection is formed by pinching the load-receiving member (10) on to the surface of the element (8).

## Revendications

1. Dispositif pour l'endoscopie médicale, comportant un élément (8, 20), en alliage superélastique, qui transmet la force et qui, en une position de couplage (9, 22, 24) formée d'au moins un évidement (13, 15, 16, 17, 18) dans la surface de l'élément, est relié, par complémentarité de forme, avec un organe de reprise de charge (10, 24), caractérisé par le fait que l'évidement (13, 15, 16, 17, 18) est formé par enlèvement de matière par sublimation.

2. Dispositif selon la revendication 1, caractérisé par le fait que l'évidement (13, 15, 16, 17, 18) est obtenu au moyen d'un rayonnement laser.

3. Dispositif selon la revendication 1, avec position de couplage (9) de forme cylindrique, caractérisé par le fait que l'évidement (13, 15, 16, 17, 18) est conçu sous forme d'un filetage (13).

4. Dispositif selon la revendication 1, caractérisé par le fait que la liaison par complémentarité de forme s'obtient par compression de l'organe de reprise de charge (10) sur la surface de l'élément (8).
